# EUROPEAN PATENT APPLICATION

(11) **EP 4 335 448 A1**
(43) Date of publication of application: **13.03.2024**
(21) Application number: 22194570.2
(22) Date of filing: 08.09.2022
(51) Int. Cl.: A61K 35/768, C12N 15/864

(54) **MODIFIED ONCOLYTIC PARVOVIRUS H-1PV WITH AN ENHANCED FITNESS AND SUPERIOR ANTICANCER ACTIVITY**

(71) Applicant: Deutsches Krebsforschungszentrum Stiftung des öffentlichen Rechts, 69120 Heidelberg (DE)
(72) Inventor: Li, Junwei, 69120 Heidelberg (DE); Autenrieth, Stella, 69120 Heidelberg (DE); Richter, Karsten, 69120 Heidelberg (DE); Hofmann, Ilse, 69120 Heidelberg (DE); Tessmer, Claudia, 69120 Heidelberg (DE)
(74) Representative: Schüssler, Andrea

(57) **Abstract**

The present invention relates to a rodent H-1 parvovirus variant capable of propagating and spreading through human tumor cells. In particular, the present invention relates to a parvovirus variant (H-1PV DR) that is based on wild-type H-1 PV but contains a 114-nucleotide internal deletion and a 55-nucleotide repeated motif towards the right-end terminus in the presence of full length of right-end ITRs. This variant displays improved anticancer activity. The present invention also relates to a pharmaceutical composition containing such a parvovirus variant as well as its use for the treatment of cancer.

## Description

The present invention relates to a rodent H-1 parvovirus variant capable of propagating and spreading through human tumor cells. In particular, the present invention relates to a parvovirus variant (H-1PV DR) that is based on wild-type H-1 PV but contains a 114-nucleotide internal deletion and a 55-nucleotide repeated motif towards the right-end terminus in the presence of full length of right-end ITRs. This variant displays improved anticancer activity. The present invention also relates to an antibody, a pharmaceutical composition and a kit containing such a parvovirus variant as well as their use for the treatment of cancer.

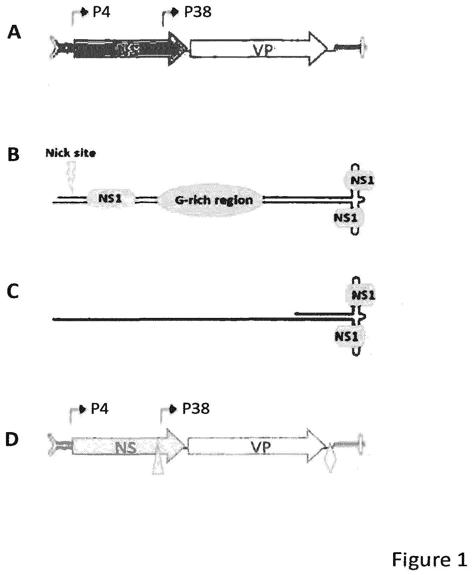

H-1 parvovirus (H-1PV), is ubiquitous, physically minute, and a genetically compact virus that contains a linear, single-stranded DNA genome of ~5 kb, which encodes only two genes that respectively code for non-structural proteins (NS) involved in the replication process and in viral cytotoxicity, and structural proteins (VP) forming the capsid (Fig.1A). The expression of viral genes is regulated by two promoters, P4 and P38. At its extremities, the viral genome contains palindromic sequences that form hairpin structures, whose size varies between the left (approx. 120 nucleotides) and the right (approx. 248 nucleotides) inverted terminal repeats (ITRs) [1].

ITRs are essential elements of the family parvoviridae including adeno-associated viruses (AAV), minute virus of mice (MVM) as well as H-1PV and are involved in the initiation of virus replication, encapsidation of its genome, excision from virus DNA replicative intermediates and long-term maintenance virus genome stability as well as transgene expression [2-4]. Rolling-circle replication is initiated by a replicon-encoded endonuclease which introduces a single-strand nick into specific origin sequences, becoming covalently attached to the 5' end of the DNA at the nick and providing a 3' hydroxyl to prime unidirectional, leading-strand synthesis [5]. In heterotelomeric parvoviruses, such as MVM and H-1PV, left-end termini in the equivalent turnaround configuration cannot be nicked, and terminal resolution is confined to the right end of the genome. A study in MVM demonstrated that virus could amplify their linear single-stranded genomes by using right ITRs that sequentially unfold and refold to shuttle the replication fork back and forth along the genome, creating a continuous, multimeric DNA strand. The viral initiator protein, NS1, then excises individual genomes from this continuum by nicking and reinitiating synthesis at specific origins present within the hairpin sequences. For nicking to occur, DNA-bending proteins from the HMGB family must coordinate interactions between the NS1 complexes bound at each end of the hairpin stem, creating a ~30-bp double-helical loop centered on the intervening G-rich region DNA [6]. There was no loop formation with mutant ITR, resulting in failure of nicking although in the presence of NS1 and HMGB, suggesting that it is critical for the activity of this hyperactive origin [7]. Since H-1PV shares a very high similarity to MVM at the right-end inverted terminal repeats, H-1PV apparently adapted the same mechanisms as MVM by using the same functional elements during viral replication (Fig. 1B) [4, 8, 9].

Wild-type H-1PV (wt H-1PV) were derived from pSR19 molecular clone. The right-end ITRs of plasmids of wild-type H-1PV (wt H-1PV) which were currently used for virus production in clinical trials, are truncated and imperfect sequences because of the original vector design for H-1PV production [9]. Nevertheless, H-1PV genomes can be replicated, even with the truncated ITRs sequences, leading to the production of H-1PV vectors in transfection experiments and further propagation in NB-324K cells.

The oncolytic virus (OV) drug "ParvOryx^{®}" utilizes the wild-type parvovirus H-1PV, which belongs to the genus Protoparvovirus within the Parvovirinae subfamily of Parvoviridae [1]. It demonstrated oncolytic and oncosuppressive properties during preclinical proof-of-concept studies in various cultured cell lines, in animal [20] and xenograft models against several human tumor species [9]. Recently, the first phase I/IIa clinical trials of "ParvOryx^{®} in patients either with recurrent glioblastoma or metastatic pancreatic carcinoma demonstrated that wt H-1PV treatment is safe, well-tolerated and associated with anticancer efficacy [22, 23]. Noticeably, there are functional elements which are absent in the wt H-1PV construct of the clinical trials. This may have a disadvantageous effect on the construct as regards virus propagation either in the virus production NB-324K cell line or human tumor cells. Thus, wt H-1PV may be improved to avoid these disadvantages.

Thus, the object of the present invention is to provide a H-1 PV construct with enhanced virus fitness and anticancer efficacy.

This technical problem is solved by providing the embodiments characterized in the independent claims. Preferred embodiments are the subject-matter of the dependent claims.

The present invention concerns the protoparvovirus (PV) H-1PV DR that is based on wild-type H-1 PV but contains a 114-nucleotide internal deletion and a 55-nucleotide repeated motif toward the right-end terminus in the presence of full length of right-end ITRs which display improved anticancer activity.

Animal viruses have the remarkable capacity to adapt to new hosts and environments [14]. The growth of natural rat parvovirus H-1PV, such as standard H-1PV (st H-1PV) in human cells frequently results in the production of variant particles with altered genomes [15-18]. Among the H-1 PV variant viruses analyzed so far, H-1 dr [internal deletion (d) and terminal reiteration (r)] is perfectly viable. H-1 dr exhibits an in-frame, 114-nucleotide deletion (nt 2022 to 2135) in the open reading frame encoding the nonstructural proteins NS1 and NS2, and a duplication of a 55-nucleotide (nt 4828 to 4883) towards the right-end terminus in viral genome (Fig. 1D).

The inventors found out that the above-mentioned H-1 dr might have an enhanced fitness after natural adaption in human cells resulting in a pronounced anticancer activity. Hence, they constructed the novel infectious recombinant molecular clone according to the featured genetic alternations in H-1 dr which was designated as H-1PV DR.

Thus, the invention describes innovative protoparvoviruses H-1PV DR featuring in a 114-nucleotide internal deletion and a 55-nucleotide repeated motif towards the right-end terminus in the presence of full length of right-end ITRs which display improved anticancer activity in different cell lines derived from various tumour entities without altering the safety profile of the virus warranting clinical translation of the new viruses into cancer patients.

The person skilled in the art can arrive at the parvovirus variant according to the present invention by subjecting a starting strain to the introduction of the before mentioned modifications resulting in the desired modification of the biological properties, i.e., capability of the parvovirus variant to propagate and spread through, thereby killing, human tumor cells starting from the known nucleic acid sequence and amino acid sequences of the non-structural proteins of parvoviruses, e.g., from parvovirus H-1 [8]. The person skilled in the art can also easily test whether a particular variant exhibits the desired biological properties recited by using the assays explained in the examples, below.

In a proof of concept study, the molecular clone of pst H-1PV (Fig.1A) was generated by inserting a full length of right-end ITRs based on H1-PV derived from pSR19 isolate [9]) and pH-1PV DR (Fig.1D) was generated by deleting a 114-nucleotides within the NS region and inserting a 55-nucleotide repeated motif toward the right-end terminus based on the plasmid of pst H-1PV. To examine whether the above modifications into the viral genome were compatible with virus life cycle and fitness, the virus production was conducted as described in Example 2 below.

The inventors examined the production and infectivity of H-1PV DR compared to those of wild-type (wt) H-1PV and standard (st) H-1PV. For this, virus was produced by infection of NB-324K cells and harvested as described in detail in Example 3 below. In these experiments, H-1PV DR demonstrated the best viral fitness in NB-324K cells which is consistent with the finding in previous observations [9].

The enhanced fitness as well as increased infectivity of H-1PV DR over wt H-1PV virions were an incentive to investigate the viral spreading efficiency in NB-324K cells during virus propagation as described in Example 3 below. The results indicated that H-1PV DR has a more efficiency of viral spreading in the process of propagation than wt H-1PV, suggesting that the new features of internal deletion and terminal reiteration of H-1PV DR might play a very important roles in the stimulation of the viral life cycle. The morphology of the various viruses was inspected by electron microscopy. Consistent with wt H1PV, H-1PV DR as well as st H1PV viruses showed the characteristic parvoviral 25-nm diameter and same morphology at high resolution.

For the sake of biosafety, in particular for future clinical application of H-1PV DR, it is worthwhile to examine the stability of either the internal deletion or terminal reiteration of H-1PV DR via consecutive infection rounds in permissive NB-324K cells. For this purpose, seven successive infection rounds were carried out. At the end of each infection round, viral genomic DNA was isolated and the genomic stability was investigated by PCR as described in Example 4 below. There was no degradation or alteration within the viral genome of H-1PV DR observed after seven rounds of infection, indicating the both modified alterations were stably integrated into the viral genome. Further sequencing of viral genome covering the modified regions demonstrated the same observations. These results are in agreement with the concept that H-1PV DR is featuring in long-term maintenance of genome stability in human cells.

The greater viral genome stability of H-1 PV DR in permissive transformed human cells, gave insight into the immune impacts and cytotoxic effect on peripheral blood mononuclear cells (PBMCs) from healthy donor, presenting the normal human cells. These experiments are described in detail in Example 5 below. It was shown that the viruses had no effects on immune cell populations and viability of PBMCs, suggesting that PBMCs are not sensitive to cytotoxic effect of H-1PV DR as well as st H-1PV, behaving similarly as wt H-1PV which has been demonstrated its perfect safety and well-tolerance of treatment in the recent completed two clinical trials [22, 23].

Although H-1PV DR showed an identical capsid assembly pattern by using electron microscopy, the occurrence of neutralizing antibodies recognition of H-1PV DR at molecular biological level because of viral genomic modifications was tested. For this purpose, neutralization assays with specific neutralizing antibodies against H-1PV DR were carried out using cytotoxicity protection assessment as described in Example 6 below. The results indicated that there are similar profiles of neutralizing antibodies recognition of H-1PV DR on capsids surfaces compared to wt H-1PV and st H-1PV, suggesting that the new features of internal deletion and terminal reiteration of H-1PV DR have no effects on viral capsids assembly pattern.

Since the phase I/IIa clinical trials in patients were conducted both in recurrent glioblastoma and metastatic pancreatic cancer by using wt H-1PV (ParvOryx^{®}), the applicant compared the anticancer effect between wt H-1PV and H-1PV DR for showing a clinical translational significance of this novel virus into new strategies of cancer treatment. As mentioned above, it was demonstrated that H-1PV DR has enhanced fitness as well as increased infectivity over wt H-1PV virions in NB-324K cells. This raised the question whether the remarkable abilities of the novel H-1PV DR are cell line dependent because of SV40 antigen which was expressed in NB-324K cells artificially [25]. Hence, the production of H-1PV DR and wt H-1PV was examined in different permissive human cell lines derived from various tumor entities as described in Example 7 below. Based on these experiments, it was ruled out that the improved fitness of H-1 PV DR was not dependent on expressing SV40 antigen in the transformed cells, indicating that H-1PV DR possesses an advantage of virus propagation in cancer cells derived from various tumor entities resulting in an excellent anticancer efficacy compared to wt H-1PV.

The enhanced virus production of H-1PV DR with potent oncolytic activities in various permissive cancer cell lines motivated to explore the efficacy of oncotoxicity of H-1 PV DR in cancer cells with different permissiveness. To this end, virus less permissive cell lines, virus semi-resistant cell lines as well as virus resistant cell lines were infected with increasing amount of virus as described in Example 8 below. Enhanced oncotoxicity of H-1PV DR was confirmed. However, it is worth noting that virus entry is a prerequisite for the production of progeny viruses resulting in oncotoxicity in cancer cells. It may explain why there is a decreasing oncolytic activity from less permissive cells to resistant cells. Importantly, H-1PV DR not only inhibited cell growth in 2D cell culture, but also in 3D spheroids of high translational relevant models featuring in more relevance to tumor biology representing the heterogeneity of cancer cells. Reference is made to Example 9 below. It can be observed that the spheroid treated with H-1 PV DR is slower in increasing of size in cancer cells compared to that with wt H-1 PV. Interestingly, there was almost retarded growth of spheroid in size over 14 days in cancer cells under treatment with H-1PV DR, suggesting that besides inhibition of proliferation there might also be a component of cell death. Altogether, these results provide proof-of-concept that H-1 PV DR has superior anticancer activity than wt H-1PV warranting clinical translation of the novel viruses into cancer patients.

As a summary, with the present invention it was possible to improve the viral fitness of H-1 PV including more efficient production as well as increased infectivity resulting in an enhanced anticancer efficacy. Remarkably, viruses harbouring the internal deletion and terminal reiteration of the present invention (H-1PV DR) showed an enhanced fitness with increased progeny production as well as augmented infectivity in comparison with st H-1PV and wt H-1PV. Importantly, H-1PV DR showed superior oncolytic activities in different cell lines derived from various tumour entities. Validation of therapeutic effects of H-1PV DR in 3D spheroids high translational relevant models confirmed the improved anticancer activity of H-1PV DR, suggesting future investigation of the novel viruses at the clinical level. In conclusion, it is the first time to demonstrate that H-1PV DR potentiates its oncolytic activity without altering the safety profiles of the virus warranting clinical translation of the new viruses into cancer patients.

Moreover, the present invention relates to antibodies which specifically recognize an above describe parvovirus variant, i.e. the polypeptide region of the parvovirus variant where the deletion is located characterizing the parvovirus variant. The antibodies can be monoclonal, polyclonal or synthetic antibodies or fragments thereof, e.g. Fab, Fv or scFV fragments. Preferably monoclonal antibodies are concerned. For the production it is favorable to immunize animals - particularly rabbits or chickens for a polyclonal antibody and mice for a monoclonal antibody - with an above parvovirus variant or with fragments thereof. Further boosters of the animals can be affected with the same parvovirus variant or with fragments thereof. The polyclonal antibody can then be obtained from the animal serum and egg yolk, respectively. The monoclonal antibody can be obtained according to standard methods, reference being made particularly to the method by Köhler and Milstein (Nature 256 (1975), 495) and Galfre (Meth. Enzymol. 73 (1981), 3). In this case, mouse myeloma cells are fused with spleen cells originating from the immunized animals. Antibodies according to the invention can be used in many ways, e.g. for the immunoprecipitation of the above described parvovirus variants or for the isolation thereof. The antibodies can be bound in immunoassays in liquid phase or to a solid carrier. In this connection, the antibodies can be labeled in various ways. The person skilled in the art is familiar with suitable markers and labeling methods. Examples of immunoassays are ELISA and RIA.

A kit is also provided for the application of the present invention. This kit comprises the following:
(a) a parvovirus variant according to the invention;
(b) an antibody according to the invention; and/or, optionally,
(c) conventional auxiliary agents, such as solvents, buffers, carriers, markers and controls.

The present invention also relates to a pharmaceutical composition comprising an effective amount of the parvovirus virus (H-1 PV DR) and/or antibody against the variant combined with a pharmaceutically acceptable carrier. The present invention also provides the use of said pharmaceutical combination for treating or preventing cancer. The present invention also relates to the use of H-1 PV DR for the preparation of (a) pharmaceutical composition(s), combination(s) or kit(s) for the treatment or prevention of cancer.

By the term "treating" and derivates thereof as used herein, is meant therapeutic therapy. In reference to a particular condition, treating means: (1) to ameliorate the condition or one or more of the biological manifestations of the conditions, (2) to interfere with (a) one or more points in the biological cascade that leads to or is responsible for the condition or (b) one or more of the biological manifestations of the condition (3) to alleviate one or more of the symptoms, effects or side effects associated with the condition, or (4) to slow the progression of the condition or one or more of the biological manifestations of the condition.

As used herein, "prevention" is understood to refer to the prophylactic administration of a drug to substantially diminish the likelihood or severity of a condition or biological manifestation thereof, or to delay the onset of such condition or biological manifestation thereof. The skilled artisan will appreciate that "preventions" is not an absolute term. Prophylactic therapy is appropriate, for example, when a subject is considered at high risk for developing cancer, such as when a subject has a strong family history of cancer or when a subject has been exposed to a carcinogen.

As used herein, the term "effective amount" means that amount of a drug or pharmaceutical agent that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder or side effect. The term also includes within its scope amounts effective to enhance normal physiological function. An "effective dose" useful for treating and/or preventing these diseases or disorders may be determined using methods known to one skilled in the art.

"Pharmaceutically acceptable" is meant to encompass any carrier, which does not interfere with the effectiveness of the biological activity of the active ingredients and that is not toxic to the patient to whom it is administered. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and can be administered to the subject at an effective dose. Additional pharmaceutically compatible carriers can include gels, bioadsorbable matrix materials, implantation elements containing the therapeutic agent, or any other suitable vehicle, delivery or dispensing means or material(s).

As used herein the term "cancer" refers to an abnormal growth of cells or tissue and is understood to include malignant neoplastic growths. The term "neoplastic" means of or related to a neoplasm. In some embodiments the cancer is a solid tumor, i.e. brain cancer (particularly gliomas: ependymomas, astrocytomas [e.g. glioblastoma multiforme], oligodendrogliomas, brainstem glioma, oligoastrocytomas); colon cancer (in particular non-MSI CRC), bladder cancer, liver cancer, breast cancer (particularly double or triple negative breast cancer), kidney cancer, head/neck squamous cell carcinoma, lung cancer (particularly lung squamous cell carcinoma, non-small-cell lung cancer (NSCLS), small-cell lung cancer (SCLC)), malignant melanoma, ovarian cancer, pancreatic cancer, prostate cancer, renal cell cancer or stomach cancer. The term "cancer" also encompasses metastasis' of the mentioned tumors in various organs. In a further preferred embodiment these tumour to be treated are recurrent tumours. A particular advantage of the pharmaceutical composition of the present invention is that even cancer initiating stem cells can be successfully treated. This has a positive effect as regards the avoidance of the recurrence of the tumours and metastasis formation.

In other embodiments the cancer is a heme malignancy, i.e. acute lymphoblastic leukemia (ALL), acute myeloid leukemia (AML), chronic lymphocytic leukemia (CLL), chronic myeloid leukemia (CML), diffuse large B-cell lymphoma (DLBCL), EBV-positive DLBCL, primary mediasinal large B-cell lymphoma, T-cell(histiocyte)-rich large B-cell lymphoma, follicular lymphoma, Hodgkin's lymphoma (HL), mantle cell lymphoma (MCL), multiple myeloma (MM), myeloid cell leukemia-1 protein (Mcl-1), myelodysplastic syndrome (MDS), non-Hodgkin's lymphoma (NHL), or small lymphocytic lymphoma (SLL).

Administration may be effected by different ways, e.g. by intravenous, intraperitoneal, subcutaneous, intramuscular, topical, intratumoral or intradermal administration. The route of administration, of course, depends on the kind of therapy and the kind of compounds contained in the pharmaceutical composition. The dosage regimen of the virus is readily determinable within the skill of the art, by the attending physician based an patient data, observations and other clinical factors, including for example the patient's size, body surface area, age, sex, the time and route of administration, the tumor type and characteristics, general health of the patient, and other drug therapies to which the patient is being subjected. Selecting a dosage regimen (also referred to herein as an administration regimen) for a therapy of the invention depends on several factors, including the serum or tissue turnover rate of the entity, the level of symptoms, the immunogenicity of the entity, and the accessibility of the target cells, tissue or organ in the individual being treated. Accordingly, the dose amount and dosing frequency depends in part on the severity of the cancer being treated, and patient characteristics.

Since the virus according to the invention comprises infectious virus particles with the ability to penetrate through the blood system, treatment can be performed (or at least initiated) by intravenous injection of the virus. Since long-term intravenous treatment is susceptible to becoming inefficient as a result of the formation of neutralizing antibodies to the virus, different modes of administration can be adopted after an initial regimen intravenous viral administration, or such different administration techniques, e.g., intratumoral virus administration, can be alternatively used throughout the entire course of viral treatment.

As another specific administration technique, the virus (virus, vector and/or cell agent) can be administered to the patient from a source implanted in the patient. For example, a catheter, e.g., of silicone or other biocompatible material, can be connected to a small subcutaneous reservoir (Rickham reservoir) installed in the patient during tumor removal or by a separate procedure, to permit the parvovirus composition to be injected locally at various times without further surgical intervention. The virus or derived vectors can also be injected into the tumor by stereotactic surgical techniques or by navigation targeting techniques.

Administration of the virus can also be performed by continuous infusion of viral particles or fluids containing viral particles through implanted catheters at low flow rates using suitable pump systems, e.g., peristaltic infusion pumps or convection enhanced delivery (CED) pumps.

A yet another method of administration of the virus is from an implanted article constructed and arranged to dispense the parvovirus to the desired cancer tissue. For example, wafers can be employed that have been impregnated with the virus, wherein the wafer is attached to the edges of the resection cavity at the conclusion of surgical tumor removal. Multiple wafers can be employed in such therapeutic intervention. Cells that actively produce the virus, or virus-based vectors, can be injected into the tumor or into the tumoral cavity after tumor removal.

In case of the parvovirus variant of the present invention the infection results in the killing of tumor cells but does not harm normal cells so that a tumor-specific therapy is achieved without adverse neurological or other side effects.

A therapy of the invention may be used prior to or following surgery to remove a tumor and may be used prior to, during or after radiation therapy.

The pharmaceutical composition may also comprise one or more additional therapeutic agents. The additional therapeutic agent may be, e.g., a chemotherapeutic agent, a biotherapeutic agent (including but not limited to antibodies to VEGF, EGFR, Her2/neu, VEGF receptors, other growth factor receptors, CD20, CD40, CD40L, CTLA-4, OX-40 4-1BB, and ICOS), a checkpoint inhibitor (e.g. antibodies against PD-1 or PD-L1), an immunogenic agent (for example, attenuated cancerous cells, tumor antigens, antigen presenting cells such as dendritic cells pulsed with tumor derived antigen or nucleic acids, immune stimulating cytokines (for example, IL-2, IFNα2, GM-CSF), and cells transfected with genes encoding immune stimulating cytokines (such as but not limited to GM-CSF). Examples of chemotherapeutic agents include alkylating agents such as cyclosphosphamide, busulfan, a camptothecin, chlorozotocin, fotemustine, lomustine, nimustine, ranimustine, antibiotics, bleomycin, caminomycin, dactinomycin, daunorubicin, idarubicin, 5-flourouracil (5-FU), methotrexate, cytarabine, platinum analog such as cisplatin and carboplatin; vinblastine, platinum; etoposide (VP-16); ifosfamide, mitoxantrone; vincristine; vinorelbine; novantrone; teniposide; edatrexate; daunomycin; aminopterin, xeloda; ibandronate; topoisomerase inhibitors; difluoromethylornithine (DMFO); retinoids, tamoxifen, raloxifene, droloxifene, 4-hydroxytamoxifen, trioxifene, keoxifene or aromatase inhibitors.

The present invention is further described with respect to the Figures which show:
**Figure 1****. Genomic structures of H-1PV DR. (A)** Schematic representation of H-1PV genome. H-1PV genome features a single line terminating in inverted terminal repeats (ITR) structures. The ITRs, sketched to illustrate the predicted structures. The viral early P4 promoter regulates the expression of the NS gene (arrowed yellow box) encoding the nonstructural NS1 and NS2, and late P38 promoter regulates the expression of the VP gene (arrowed light yellow box) encoding the VP1 and VP2 viral proteins. The right-end ITRs is supposed to serve as self-priming origins of virus genome replication and encapsidation. **(B)** Anticipated structure of the right-end ITRs of MVM and H-1 PV. The 248-nucleotide right-end telomere presents in black line. A 36-bp palindrome containing reiterations of the NS1-binding motif, 5'-TGGT-3' (small light orange box), folds into two axial arms. The assembly of NS1 oligomers bind the boxed 5'-TGGT-3' motifs in the hairpin stem is illustrated by larger light orange box. The nick site is indicated by yellow lightning bolt and G-rich region by green oval. **(C)** Truncated structure of the right-end hairpin of H-1PV. The 182-nucleotide right-end ITRs of H-1PV is shown as black line. A 36-bp palindrome containing reiterations of the NS1-binding motif, 5'-TGGT-3', folds into two axial arms is indicated by small light orange box. **(D)** Diagrammatic representation of H-1PV DR genomic organization. The grey triangle indicates the position of 114-nucleotide internal deletion and the pink diamond shows the location of a 55-nucleotide repeated motif toward the right-end terminus. The rest features of viral genome were described as **(A).**

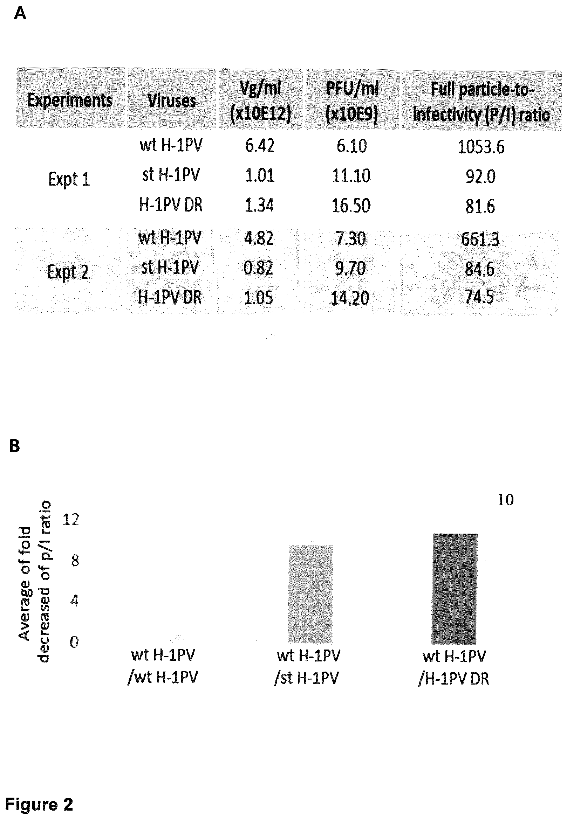

**Figure 2****. Increased progeny production and infectivity of H-1PV DR with an enhanced fitness in permissive human NB-324K cells. (A)** Increased infectivity of H-1PV DR compared to wt H-1PV as well as st H-1PV. Cells infected with indicated virus at a MOI 0.01 PFU per cell were harvested at day 5 post-infection. Titers of infectious particles as well as genome-containing particles were determined by plaque assay (given in PFU/ml) and qPCR (given in vg/ml), respectively. The genome-containing particle-to-infectivity (P/I) ratio was shown in the table. **(B)** The fold decrease of the P/I ratio between wt H-1PV versus st H-1PV as well as wt H-1PV versus H-1PV DR are exhibited.

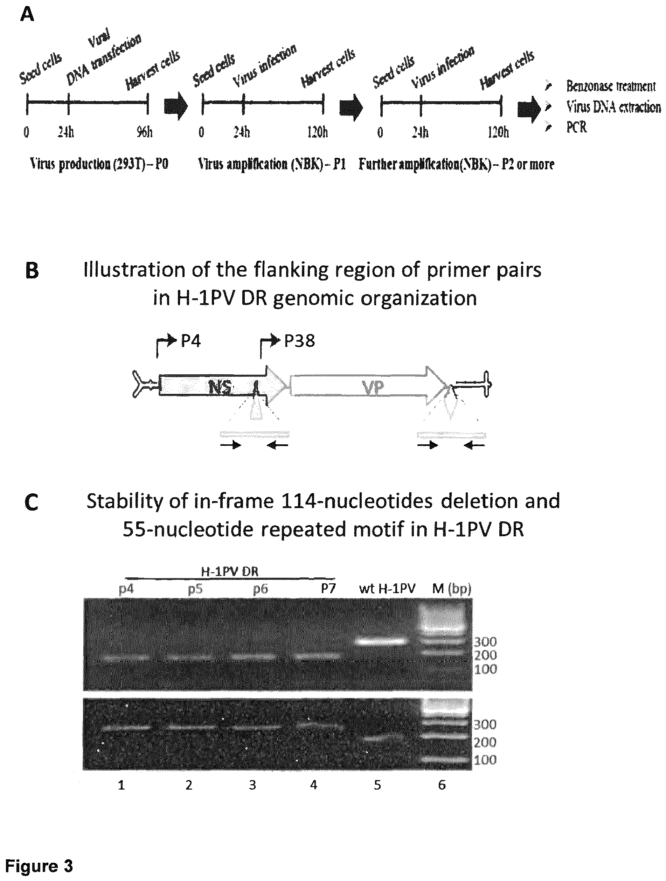

**Figure 3****. Evaluation of genomic stability of H-1PV DR. (A)** Production of H-1PV DR via consecutive infection rounds. The production scheme was adopted for checking the stability of the new features of internal deletion and terminal reiteration of H-1PV DR. Transfection of H-1 PV DR in HEK293T cells results in generation of fully infectious virus particles. NB-324K cells were infected with indicated viruses used at a MOI of 0.01 PFU per cell. After 5 days, infected cells were collected and the progeny virus particles were purified, titrated and used to infect a freshly prepared batch of cells. Each round of infection and harvesting corresponds in the figure to one passage. i.e. one round of virus production (P); Seven rounds of production were carried out (from P1 to P7). From each round of production, virus DNA was extracted and the genomic fragment including the internal deletion and terminal reiteration of H-1PV DR was amplified by PCR. **(B)** Illustration of the flanking region of primer pairs in H-1PV DR genomic organization. **(C)** Stability of in-frame 114-nucleotides deletion (upper panel) and 58-nucleotide repeated motif (lower panel) in H-1PV DR was assessed by PCR analysis.

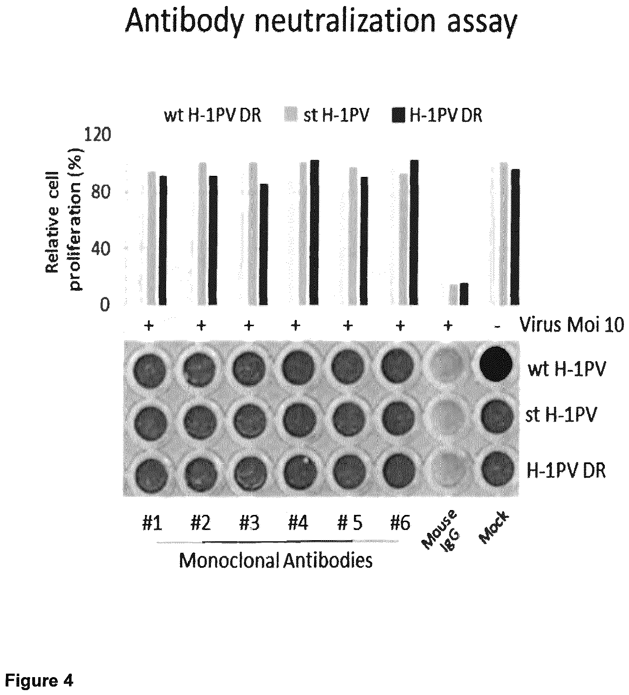

**Figure 4****. Evaluation of profiles of neutralizing antibody recognition of H-1PV DR on capsids surfaces.** Neutralization assay of indicated viruses by specific neutralizing antibodies against H-1PV DR as described in the Materials and Methods section. The activity of virus neutralizing antibodies were determined using cytotoxicity protection assessment by Cell Counting Kit-8 assays on NB-324K cells at a MOI 10 PFU per cell scored after 72h post-inoculation (upper panel). Effects of neutralizing antibodies on NB-324K cells by using same conditions as described above were demonstrated with crystal violet staining assays independently (lower panel).

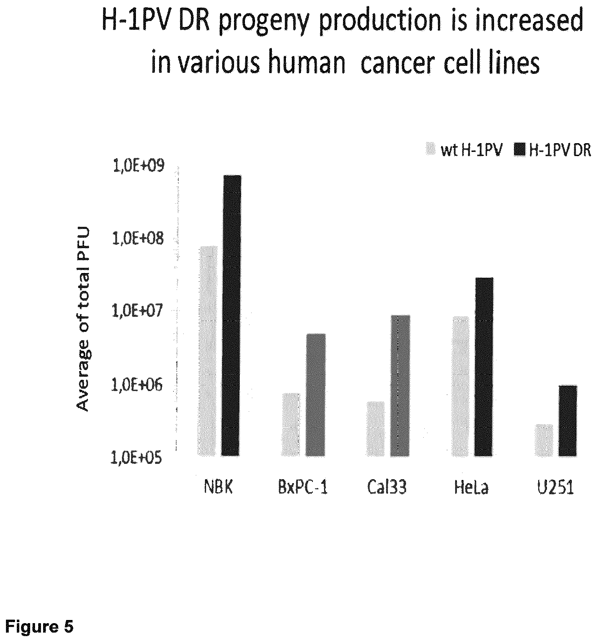

**Figure 5****. The more efficient production of H-1PV DR is not only limited in NB-324K cells.** H-1PV DR progeny production was measured in various human cell lines by plaque assays. Infectious progeny production of H-1PV DR and wt H-1PV was measured after infection at MOI 0.25 PFU per cell in human NB-324K, BxPC-1, Cal33, Hela and U251 cell lines. The yields of infectious particles (total numbers of PFU) recovered from both cell supernatants and pellets were determined by plaque assay at days 3 post-infection for NB-324K and days 4 for the rest of cells lines. The results represented the average of two independent experiments.

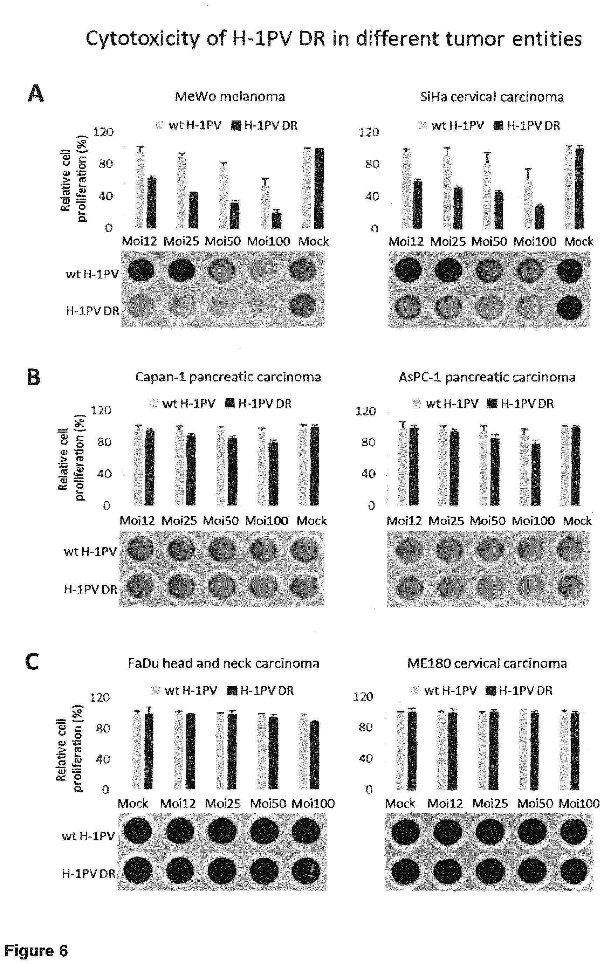

**Figure 6****. H-1PV DR has superior oncolytic activity in various human cell lines derived from different cancer entities** Cell proliferation and viability assessment by Cell Counting Kit-8 assays as well as crystal violet staining assays respectively were conducted upon infection of H-1PV DR and wt H-1PV with increasing amounts of MOI from 12 to 100 PFU per cell at 96h post-infection. **(A)** Virus less permissive cell lines MeWo and SiHa. **(B)** Virus semi-resistant cell lines Capan-1 and AsPC-1. **(C)** Virus resistant cell lines FaDu and ME180.

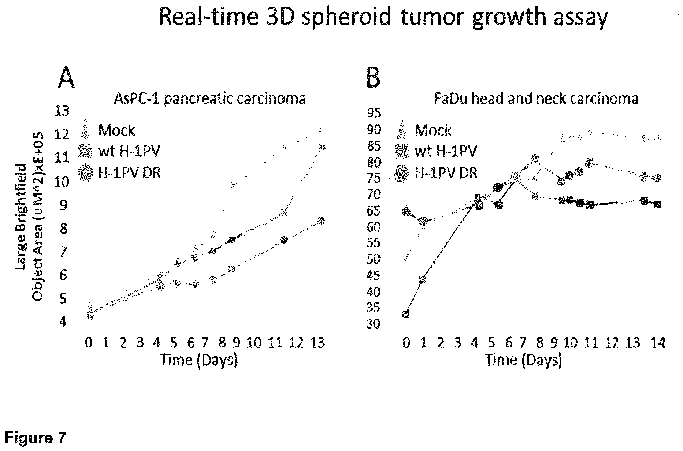

**Figure 7****. Validation of therapeutic effects of H-1PV DR in 3D spheroids high translational relevant models.** Cell real time proliferation by Incucyte^{®} single spheroid assay was documented upon infection of H-1 PV DR and wt H-1PV at a MOI 10 PFU per cell till 13 or 14 days post-infection. **(A)** Virus semi-resistant cell line AsPC-1. **(B)** Virus resistant cell line FaDu.

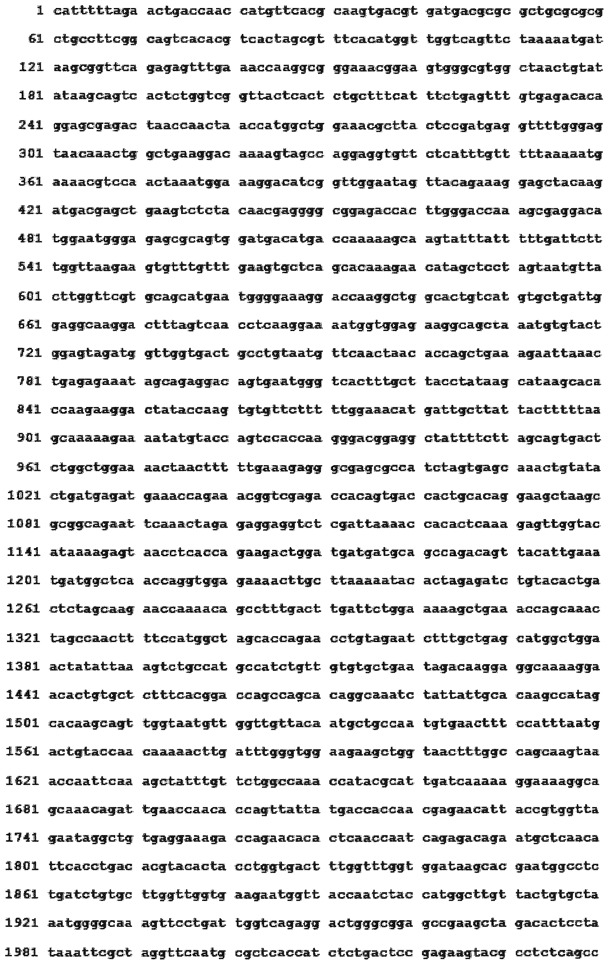

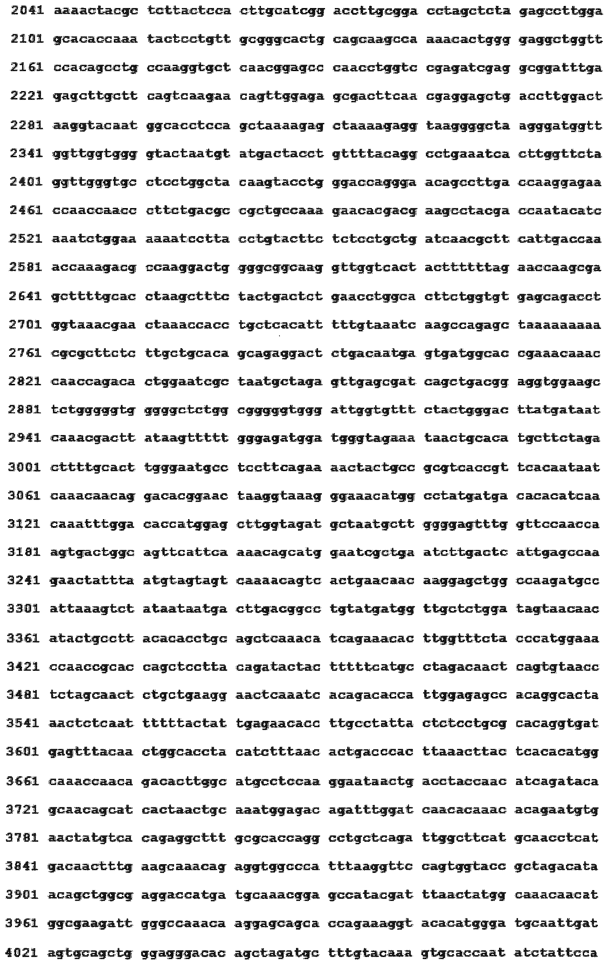

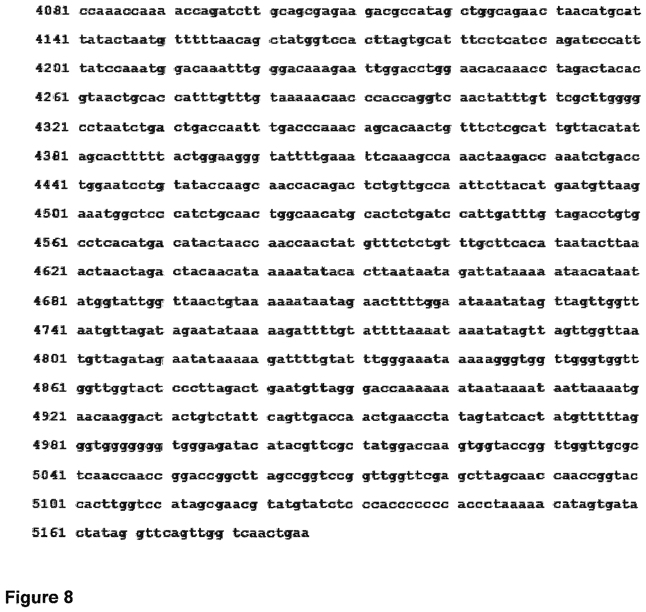

**Figure 8****. Sequence of Parvovirus H1**

The invention is further described in the following Examples. These Examples are to be understood as preferred embodiments but are not intended to limit the invention.

### Example 1: Materials and Methods

**Plasmid construct.** For the construction of pst H-1PV, a fragment of the viral genome obtained by digesting the pSR19 clone with *Hpal-Ndel,* was first sub-cloned into the pUC 19 *Hpal-Ndel* vector, generating an adapting vector with a modified polylinker. The DNA fragment containing a full-length right-end ITRs flanking with *PshAl* and *Ndel* restriction sites synthetically manufactured by Biocat (Heidelberg, Germany) was shuttled into this vector, resulting in the construct of pst H-1PV (*Hpal-Ndel*)*.* Using the same strategy, the DNA fragment containing a 55-nucleotide repeated motif (nt4828 to 4883) flanking with *Hpal* and *PshAl* restriction sites was introduced into pstH-1PV (*Hpal-Ndel*)*.* The modified constructs finally were sub-cloned back into their parental pSR19 backbone, generating the molecular clones pst H-1PV and pH-1PV R. For construction of H-1 PV DR, the deletion presenting an in-frame 114 nucleotides (nt2022 to 2135) within the NS region in plasmid of pDelH1 digested with *EcoRl-Mfel* was introduced into vector of pH-1PV R [19]. The resulted in the pst H-1PV and pH-1PV DR plasmids in which the substituted fragments were further verified by sequencing (Genewiz, Takeley Sanger Sequencing Laboratory, United Kingdom).

**Cell cultures.** Simian virus 40 (SV40)-transformed human newborn kidney NB-324K cells and HEK293T cells were as described previously [25, 26]. The human glioblastoma (GBM) cell line U251, was a kind gift of Dr. Iris Augustin (DKFZ, Heidelberg, Germany). HeLa and SiHa cervical carcinoma (CC) cell lines were kindly provided by Dr. Angel Alonso (DKFZ, Heidelberg, Germany). ME180 cervical carcinoma (CC) cell line were kindly provided by Dr. Elisabeth Schwarz (DKFZ, Heidelberg, Germany). The pancreatic ductal adenocarcinoma (PDAC) derived cell lines AsPC-1, BxPC-1 and Capan-1 were kindly provided by Dr. Stephan Herzig (DKFZ, Heidelberg, Germany). The head and neck squamous cell carcinomas (HNSCCs) cell lines FaDu and Cal33 were kindly provided by Dr. Ina Kurth (DKFZ, Heidelberg, Germany). The melanoma MeWo cell line is a kind gift of Dr. Jochen Utikal (DKFZ, Heidelberg, Germany). Cells were cultivated in media according to Table 1, supplemented with 10% fetal bovine serum (FBS), except for NB-324K cells with 5% FBS (Sigma Aldrich), 2 mM L-glutamine (Gibco) and antibiotics (100 U/ml of penicillin and 100 µg /ml of streptomycin sulfate, Gibco). All cells were grown at 37°C, 5% CO₂, 95% humidity and routinely checked for mycoplasma contamination using the Mycoplasma Detection Kit according to the manufacturer's instructions (Venor GeM, Minerva Biolabs, Berlin, Germany).

**Table 1: Cell lines and corresponding media and supplements (where applicable)**

| Cell line | Medium | Supplement 1 | Supplement 2 | Supplement 3 |
|---|---|---|---|---|
| HEK293T | DMEM | | | |
| NB-324K | MEM | | | |
| U251 | DMEM | | | |
| HeLa | DMEM | | | |
| SiHa | DMEM | | | |
| ME180 | DMEM | | | |
| AsPC-1 | DMEM | | | |
| BxPC-1 | DMEM | | | |
| Capan-1 | DMEM | | | |
| FaDu | DMEM | 100mM Sodium Pyruvat | 1M Hepes | non essential amino acid |
| Cal33 | DMEM | | | |
| MeWo | DMEM | | | |
| PBMC | RPMI1640 | | | |

For experiments, cells were counted using trypan blue and CountessTM Automated Cell Counter (Invitrogen, USA) and seeded at the cell numbers given in Table 2 if not stated otherwise. 3 ml medium was used for 6-well plate, 5 ml medium for 6 cm dish, and 100 µl/well for 96-well plates.

**Table 2: Cell numbers seeded for experiments**

| Cell line | 6 cm dish | 6-well plate (per well) | 96-well plate (per well) |
|---|---|---|---|
| HEK293T | | 8×10⁵ | |
| NB-324K | 5×10⁵ | | 4000 |
| U251 | 5×10⁵ | | |
| HeLa | 5×10⁵ | | |
| SiHa | | | 4000 |
| ME180 | | | 8000 |
| AsPC-1 | | | 4000 |
| BxPC-1 | 5×10⁵ | | |
| Capan-1 | | | 8000 |
| FaDu | | | 4000 |
| Cal33 | 5×10⁵ | | |
| MeWo | | | 4000 |
| PBMC | | 1×10⁶ | |

**Transfection assays.** HEK293T cells were seeded in 6 well plate with 3ml of Opti-MEM reduced serum medium (Invitrogen, Cat. No. 31985). On the next day, cells were transiently transfected with 6 µg of plasmids harbouring the virus genome (pwt H-1PV, pstH-1PV or pH-1PV DR) using the transfection reagent Metafectene (Biontex Laboratories GmbH, Cat. No. T020-1.0, Munich, Germany) at 1:2 ratio (µg DNA: µl reagent) according to manufacturer's instructions.

**Virus infection and production.** The above viruses were primarily produced by transfection of 293T cells and subsequently amplified by infection of NB-324K cells at a multiplicity of infection (MOI) of 0.01 PFU per cell. The harvest of cells and purification of viruses by iodixanol step gradient centrifugation according to the methods described previously [27].

**Titration of infectious and full particles.** Infectious viral particles were titrated by plaque assay as previously described [25]. Full viral particles were quantified by quantitative real-time PCR as described previously [28]. Virus titers were expressed as the number of viral genomes (vg) per milliliter of virus stock.

**Electron microscopy.** Viruses from dialysed preparations were adsorbed onto glow discharged carbon coated grids, washed with water (Braun, Ampuwa) and negatively stained with 1% aqueous uranyl acetate. Micrographs were acquired using an EM 912 at 80kV (Carl Zeiss Company, Oberkochen, Germany) equipped with a slow scan CCD camera (TRS, Moorenweis, Germany).

**Evaluation of the stability of internal deletion and terminal reiteration by PCR.** NB-324K cells were infected with H-1PV DR via consecutive rounds. At the end of each infection round, viral genomic DNA was isolated as previously described [26]. The PCR were performed by using CloneAmp HiFi PCR Premix (Takara Bio Clontech. Cat. No. 639298, Japan) with primers pairs (Deletion For 5'-TCAATGCGCTCACCATCTCTG-3' versus Deletion Rev 5'-TTAGTCCAAGGTCAGCTCCTC -3' or Reiteration For 5'-TAATATGGTATTGGTTAACTGTAAAAAAT -3' versus Reiteration Rev 5'-CAACCACCCAACCACCCTTT - 3'). The PCR mixture was loaded into 2% or 3% agarose gel for electrophoresis. Images were documented by INTAS (Intas Pharmaceuticals Limited, Indian).

**Spectral Flow cytometry data analysis.** PBMCs were stained in PBS for 30 min at room temperature in the dark with a 1:1000 dilution ZombieNIR as life dead stain. Thereafter, FcX and monocyte blocker (Biolegend) were added for 10 min at room temperature in the dark followed by addition of fluorchrome labeled antibodies (Table 1). After incubation of 60 min at room temperature in the dark, cells were washed and measured on an AURORA spectral flow cytometer (Cytek Biosciences). Unsupervised data analysis as detailed below was done using OMIQ data analysis software (www.omiq.ai). First, the data were manually gated to remove aggregates, dead cells, debris, and then the data were sub-sampled to include 2.5×10⁵ CD45⁺ leukocytes/group. Next, flowAI was run to check for any aberrant regions of the files [29]. FlowAI settings were as follows: all files used, all fluorescent channels and time selected, all methods used and default settings. Subsequently, dimension reduction analysis was performed using Uniform Manifold Approximation and Projection (UMAP) to visualize the different sub-populations in groups [30]. UMAP settings were as follows: all files used, all fluorescent parameters were used besides CD45 and Live/Dead, Neighbors = 80, Minimum Distance = 0.7, Components = 2, Metric = Euclidean, Learning Rate = 1, Epochs = 250, Random Seed = 9346, Embedding Initialization = spectral.

**Development of monoclonal antibodies.** Generation of monoclonal antibodies was conducted according to the principles of technology [31]. For mouse immunization, several injections were performed using virus particles H-1PV DR. To enhance the immune response, 100 µl of Freund's Complete Adjuvant (Santa Cruz Biotechnology) were injected in the hind leg of each mouse. Boost injections were done with Freund's Incomplete Adjuvant followed by injection with buffer only. The fusion procedure to originate hybridoma cell clones was carried out as follows: popliteal lymph nodes were surgically removed and placed in RPMI medium (Gibco). Lymph nodes were subsequently ground with a syringe plunger under the microscope. The cell mixture was centrifuged at 150xg for 10 min at room temperature. Cells were resuspended in 1.5 ml polyethylene glycol (PEG, Sigma- Aldrich) was added over 1 min and mixed with a Pasteur pipette. Successively 20 ml RPMI medium were added over 4 min and cells were centrifuged at 150xg for 10 min and later resuspended in HAT medium containing Hyper and cultured for 7 days. After 7 days cell supernatant was screened for the presence of specific antibodies to the protein of interest by ELISA followed by immunofluorescence. Validated mother clones were subcloned by limited dilution to obtain monoclonal cell clones.

**Neutralization assay.** Virions (10 PFU per cell) were incubated with different clonal antibodies against H-1PV DR in final volume at 60 µl of primary MEM medium for 30 min at 37°C. The remaining infectivity was determined on NB-324K cells after 72h post-inoculation using Cell Counting Kit-8 assays as well as crystal violet staining assays.

**Cell Counting Kit-8 assay.** The cells were seeded in 96-wells plate containing 50 µl/well of medium. Their respective culture media and cell numbers were according to Table1 and 2, namely NB-324K, MeWo, SiHa, AsPC-1, Canpan-1, FaDu and ME180. After 24 hours, 50 µl/well of FBS-free medium with or without st H-1PV or H-1PV DR was added. After 72 or 96 hours post-infection, the cell proliferation were performed using Cell Counting Kit-8 assay (Dojindo, Kumamoto, Japan) as previously described [32].

**Crystal violet staining assay.** The seeding of cells as well as the treatment were described as above. The cell viability were performed using crystal violet assay as previously described [33].

**Generation of spheroids.** 3D cell spheroids represented the heterogeneity of a tumor model because cells in the outer layer of the spheroid have access to nutrients and oxygen, whereas in the core of the spheroid a hypoxic region forms by accumulation of degraded products of cells. Spheroids were created from 20000 AsPC-1 or Fadu cells using hanging drop method in the presence of 30% methylcellulose stock solution as previously described [34]. The formed spheroids after 2-3 days were transferred to a low attachment round-bottom 96-well plates. 50 µl/well of complete medium with or without st H-1PV or H-1PV DR was added. The size of spheroid was analyzed in real-time with the Incucyte^{®} 3D Single Spheroid Assays using the acquisition and analysis tool for spheroids. Usually, 3 images per well were acquired every day at a magnification of 10x. Analysis was performed with the IncuCyte^{®} S3 2018A software.

### Example 2: Generation and production of H-1PV DR

In a proof of concept study, the molecular clone of pst H-1PV (Fig.1A) was generated by inserting a full length of right-end ITRs based on the previous plasmid of pSR19 [9] and pH-1PV DR (Fig.1D) was generated by deleting a 114-nucleotides within the NS region and inserting a 55-nucleotide repeated motif toward the right-end terminus based on the plasmid of pst H-1PV. To examine whether the above modifications into viral genome were compatible with virus life cycle and fitness, the virus production was conducted. Routinely, parvovirus production is firstly performed in HEK293T cells by transient transfection of the construct of pst H-1PV and pH-1PV DR. About 96 hours post-transfection, cells are lysed and newly assembled viral particles purified and titrated by plaque assay and quantitative real-time PCR. Virus further amplification was carried out in NB-324K cells. Cells are infected at a MOI 0.01 PFU (plaque forming unit/ml) per cell by using the virus previously produced in transfection. When cytopathic effects indicating for virus replication and egress are detected in 80% of the cells, cells are harvested and virus are purified and titrated. As expected, viruses were successfully purified from cell lysates. For further studies, wt H-1PV designated pSR19, st H-1PV and H-1PV DR were transfected into HEK293T cells to generate master stocks respectively. Individual virus was produced in parallel by infection of NB-324K cells as described in Example 1 above.

### Example 3: Increased progeny production and infectivity of H-1PV DR with an enhanced fitness in permissive human NB-324K cells.

The inventors examined the production and infectivity of H-1PV DR compared to those of wild-type (wt) H-1PV and standard (st) H-1PV. For this, virus were produced at the same time by infection of NB-324K cells and harvested at 72 hours post-infection. The amounts of genome-containing (i.e., full) and plaque-forming (i.e., infectious) particles were determined by quantitative real-time PCR and plaque assay, respectively. The amounts of full (given in vg/ml) viral particles and the titers of infectious (given in PFU/ml) from two independent experiments are listed in Fig. 2A. Although the amounts of full particles produced by infection with wt H-1PV were quite higher than those with st H-1PV and H-1PV DR, the later two yielded higher titers of infectious virions than wt H-1PV. Interestingly, there are dramatically decreased ratios of the corresponding full particle-to-infectivity (P/I) as shown in Fig. 2A right column, resulting in a significantly (8- to 10-fold) lower P/I ratio with st H-1PV as well as H-1PV DR (Fig. 2B). These results suggested that the full length of right-end ITRs are key elements of parvovirus involved in encapsidation of viral genome, excision from virus DNA replicative intermediates, leading to a high yield of infectious virions. Last but not least, H-1PV DR demonstrated with the best viral fitness in NB-324K cells and it is consistent with the finding in previous observations [9].

The enhanced fitness as well as increased infectivity of H-1PV DR over wt H-1PV virions were an incentive to investigate the viral spreading efficiency in NB-324K cells during virus propagation. To this end, plaque assays were performed. All viruses generated a mixture of small and large plaques. Distinctly there is a higher frequency of large plaques in the cells infected with H-1PV DR, whereas a higher frequency of small plaques was obtained with wt H-1PV. These results indicated that H-1PV DR has a more efficiency of viral spreading in the process of propagation than wt H-1PV, suggesting that the new features of internal deletion and terminal reiteration of H-1 PV DR might play very important roles in the stimulation of the viral life cycle. The morphology of the various viruses was inspected by electron microscopy. Consistent with wt H1PV, H-1PV DR as well as st H1PV viruses showed the characteristic parvoviral 25-nm diameter and same morphology at high resolution.

### Example 4: Evaluation of the stability of internal deletion and terminal reiteration of H-1PV DR

For the sake of biosafety, in particular for future clinical application of H-1PV DR, it is worthwhile to examine the stability of either the internal deletion or terminal reiteration of H-1PV DR via consecutive infection rounds in permissive NB-324K cells. For this purpose, seven successive infection rounds were carried out as indicated in Fig. 3A. At the end of each infection round, viral genomic DNA was isolated and the genomic stability was investigated by PCR using primers flanking the region either at position of nucleotides 2022 to 2135 or nucleotides 4828 to 4883 as shown in Fig. 3B.

There was no degradation or alteration within viral genome of H-1PV DR observed after seven rounds of infection, indicating the both modified alterations were stably integrated in the viral genome as shown in Fig. 3C from lane 1 to 4 representing the viral passage from 4 to 7 correspondingly. The unmodified wt H-1PV was used as control (Fig. 3C, lane 5). Further sequencing of viral genome covering the modified regions demonstrated the same observations. These results are in agreement with the concept that H-1PV DR is featuring in long-term maintenance of genome stability in human cells.

### Example 5: Non-Sensitivity of normal human cells to H-1PV DR cytotoxic effect

The greater viral genome stability of H-1 PV DR in permissive transformed human cells, gave insight into the immune impacts and cytotoxic effect on peripheral blood mononuclear cells (PBMCs) from healthy donor, presenting the normal human cells. To this end, spectral flow cytometry assay was performed upon infection PBMCs ex *vivo* cultured with wt H-1PV, st H-1PV and H-1PV DR respectively at the highest MOI of 10 PFU per cell. The analysis of the data was conducted by using the 36-color panel for immune-phenotyping. UMAP plot showed highly overlapping immune cell populations with any condition analyzed including mock-infected PBMCs, demonstrating no influence of virus infection on any immune cell population under the challenging with infection of viruses. The viruses had no effects on immune cell populations and viability of PBMCs, suggesting that PBMCs are not sensitive to cytotoxic effect of H-1PV DR as well as st H-1PV, behaving similarly as wt H-1PV.

### Example 6: Evaluation of profiles of neutralizing antibodies recognition of H-1PV DR on capsids surfaces

Although H-1PV DR showed an identical capsid assembly pattern by using electron microscopy, the occurrence of neutralizing antibodies recognition of H-1PV DR at molecular biological level because of viral genomic modifications was tested. For this purpose, neutralization assays with specific neutralizing antibodies against H-1PV DR were carried out using cytotoxicity protection assessment. The activity of virus neutralizing antibodies were determined by Cell Counting Kit-8 assays on NB-324K cells at a MOI 10 PFU per cell scored after 72 hour post-inoculation (Fig. 4, upper panel). As a control, mouse IgG showed no activity of virus neutralizing, resulting in failure of cytotoxicity protection after incubation with viruses. On the contrary, the different monoclonal antibodies against H-1PV DR revealed pronounced abilities to neutralize the infectivity of virus, generating a potent cytotoxicity protection as mock-infected NB-324K cells. Interestingly, the same observations were found when the experiments were conducted by using wt H-1PV and st H-1PV under the same set of experimental settings. The similar effects of neutralizing antibodies on various viruses in NB-324K cells by using same conditions were demonstrated with crystal violet staining assays independently (Fig. 4, lower panel). These results indicated that there are similar profiles of neutralizing antibodies recognition of H-1PV DR on capsids surfaces compared to wt H-1PV and st H-1PV, suggesting that the new features of internal deletion and terminal reiteration of H-1PV DR have no effects on viral capsids assembly pattern.

### Example 7: Efficient production of H-1PV DR in different permissive human cancer cell lines

As mentioned above, it was demonstrated that H-1PV DR has enhanced fitness as well as increased infectivity over wt H-1PV virions in NB-324K cells. This raised a question that if the remarkable abilities of the novel H-1PV DR are cell line dependent because of SV40 antigen which was expressed in NB-324K cells artificially [25]. Hence, the production of H-1PV DR and wt H-1PV was examined in different permissive human cell lines derived from various tumor entities. Cells of NB-324K, pancreatic ductal adenocarcinoma (PDAC) BxPC-1, head and neck squamous cell carcinomas (HNSCCs) Cal33, cervical carcinomas HeLa and glioblastoma (GBM) U251 were infected at a MOI 0.25 PFU per cell with H-1PV DR as well as wt H-1PV. The infectious virions were harvested at days 3 post-infection for NB-324K cells and days 4 for the rest of cell lines and quantified by plaque assays. The amount of total (combined cell pellets and medium) infectious particles was not only higher in NB-324K cells upon infection with H-1PV DR than wt H-1PV, but also increased in the rest of four cell lines from different tumor entities (Fig. 5). Based on these observations, it was ruled out that the improved fitness of H-1PV DR was not SV40 antigen-expressing dependent in the transformed cells, indicating that H-1PV DR possesses an advantage of virus propagation in cancer cells derived from various tumor entities resulting in an excellent anticancer efficacy compared to wt H-1PV.

### Example 8: Superior oncotoxicity of H-1PV DR than wt H-1PV

The enhanced virus production of H-1PV DR with potent oncolytic activities in various permissive cancer cell lines motivated the inventors to explore the efficacy of oncotoxicity of H-1PV DR in cancer cells with different permissiveness. To this end, virus less permissive cell lines including melanoma MeWo and cervical carcinoma SiHa (Fig. 6A), virus semi-resistant cell lines including pancreatic ductal adenocarcinoma (PDAC) Capan-1 and AsPC-1 (Fig. 6B) as well as virus-resistant cell lines including head and neck squamous cell carcinomas (HNSCCs) FaDu and cervical carcinoma ME180 (Fig. 6C) were infected with increasing amount of virus (MOI from 12 to 100 PFU per cell) of H-1PV DR, wt H-1PV or treated with virus-dilution buffer as mock. Four days post-infection, virus-induced cytotoxicity was determined by analysing cell proliferation using cell Counting Kit-8 assays. As shown in Fig. 6A, upper panel, dramatically decreased cell proliferation was observed in both of cell lines treated with H-1PV DR compared to wt H-1PV in a virus dose-dependent manner. Enhanced oncotoxicity of H-1PV DR was also confirmed by analysing the cells viability using crystal violet staining assays (Fig. 6A, lower panel). However, the decreased cell proliferation as well as viability in semi-resistant cell lines, either Capan-1 or AsPC-1, were only observed at higher titers of virus and the stronger inhibition of cell growth were observed in the cells treated with H-1PV DR (Fig. 6B). Furthermore, there are not any effects of wt H-1PV on virus resistant cell lines either FaDu or ME180 cells, even at highest titers of virus. Slightly only decreased cell proliferation was observed in FaDu cells treated with H-1PV DR at highest titers of virus (Fig. 6C). It is worth noting that virus entry is a prerequisite for the production of progeny viruses resulting in oncotoxicity in cancer cells. It may explain why there is a decreasing oncolytic activity from less permissive cells to resistant cells.

Importantly, H-1PV DR not only inhibited cell growth in 2D cell culture, but also in 3D spheroids of high translational relevant models featuring in more relevance to tumor biology representing the heterogeneity of cancer cells. For the generation of spheroids a protocol was established which includes forming spheroids by hanging drop using supplementing the medium with methylcellulose before transferring in an ultra-low attachment microplate. The size of spheroids upon treatment with H-1PV DR and wt H-1PV at a MOI 10 PFU per cell was measured with Incucyte^{®} 3D Single Spheroid Assays using the acquisition and analysis tool for spheroids. This device is able to monitor cell proliferation in real time. Figure 8 A and B depict the increase of spheroids size upon treatment. It can be observed that the spheroid treated with H-1PV DR is slower in increasing of size in AsPC-1 cells compared to that with wt H-1PV (Fig. 7A). Interestingly, there was almost retarded growth of spheroid in size over 14 days in FaDu cells under treatment with H-1 PV DR (Fig. 7B), suggesting that besides inhibition of proliferation there might also be a component of cell death. Altogether, these results provide proof-of-concept that H-1PV DR has superior anticancer activity than wt H-1PV warranting clinical translation of the novel viruses into cancer patients.

### References:

1. Cotmore, S. F. and Tattersall, P. (2014) Parvoviruses: small does not mean simple. Annu. Rev. Virol. 1, 517-537
2. Savy A, Dickx Y, Nauwynck L, Bonnin D, Merten OW, Galibert L. (2017) Impact of Inverted Terminal Repeat Integrity on rAAV8 Production Using the Baculovirus/Sf9 Cells System. Hum Gene Ther Methods. 28, 277-289
3. Willwand K, Baldauf AQ, Deleu L, Mumtsidu E, Costello E, Beard P, Rommelaere J. (1997) The minute virus of mice (MVM) nonstructural protein NS1 induces nicking of MVM DNA at a unique site of the right-end telomere in both hairpin and duplex conformations in vitro. J Gen Virol. 78, 2647-55
4. Rhode SL 3rd, Klaassen B. (1982) DNA sequence of the 5' terminus containing the replication origin of parvovirus replicative form DNA. J Virol. 41, 990-999
5. Tattersall P, Ward DC. (1976) Rolling hairpin model for replication of parvovirus and linear chromosomal DNA. Nature 263, 106-109
6. Cotmore SF, Tattersall P. (1998) High-mobility group 1/2 proteins are essential for initiating rollingcircle- type DNA replication at a parvovirus hairpin origin. J. Virol. 72, 8477-8484
7. Cotmore SF, Christensen J, Tattersall P. (2000) Two widely spaced initiator binding sites create an HMG1-dependent parvovirus rolling-hairpin replication origin. J Virol. 74, 1332-1341.
8. Rhode SL 3rd, Paradiso PR. (1983) Parvovirus genome: nucleotide sequence of H-1 and mapping of its genes by hybrid-arrested translation. J Virol. 45, 173-184
9. Faisst S, Faisst SR, Dupressoir T, Plaza S, Pujol A, Jauniaux JC, Rhode SL, Rommelaere J. (1995) Isolation of a fully infectious variant of parvovirus H-1 supplanting the standard strain in human cells. J Virol. 69, 4538-4543
10. Yan Z, Zak R, Zhang Y, Engelhardt JF. (2005) Inverted terminal repeat sequences are important for intermolecular recombination and circularization of adeno-associated virus genomes. J Virol. 79, 364-379
11. Zhou Q, Tian W, Liu C, Lian Z, Dong X, Wu X. (2017) Deletion of the B-B' and C-C' regions of inverted terminal repeats reduces rAAV productivity but increases transgene expression. Sci Rep. 7, 5432.
12. Colella P, Ronzitti G, Mingozzi F. (2017) Emerging Issues in AAV-Mediated In Vivo Gene Therapy. Mol Ther Methods Clin Dev. 1, 87-104
13. Willwand K, Mumtsidu E, Kuntz-Simon G, Rommelaere J. (1998) Initiation of DNA replication at palindromic telomeres is mediated by a duplex-to-hairpin transition induced by the minute virus of mice nonstructural protein NS1. J Biol Chem. 273, 1165-1174
14. Simmonds P, Aiewsakun P, Katzourakis A. (2019) Prisoners of war - host adaptation and its constraints on virus evolution. Nat Rev Microbiol 17, 321-328
15. Carter, B. J. (1984) Variant and defective interfering parvoviruses. In K. I. Berns (ed.). The parvoviruses. p209-258
16. Faust, E. A., and A. Hogan. (1990) Defective interfering particles. In P. Tijssen (ed.). Handbook of parvoviruses. p91-107
17. Rhode, S. L. (1978) Defective interfering particles of parvovirus H-1. J. Virol. 27, 347-356
18. Rhode SL 3rd. (1978) Replication process of the parvovirus H-1. X. Isolation of a mutant defective in replicative-form DNA replication. J Virol. 25, 215-223
19. Weiss N, Stroh-Dege A, Rommelaere J, Dinsart C, Salomé N. (2012) An in-frame deletion in the NS protein-coding sequence of parvovirus H-1PV efficiently stimulates export and infectivity of progeny virions. J. Virol. 86, 7554-7564
20. Nuesch J., Thomas, N., Plotzky, C., Rommelaere, J. (2011) Modified rodent parvovirus capable of propagating and spreading through human gliomas. PCT/EP201 1/002306
21. Thomas, N. (2011) Therapie von Hirntumoren (Gliomen) mit dem onkolytischen Parvovirus H-1 (H-1PV) Präklinische Untersuchungen zur Optimierung der Virotherapie in vitro und in vivo. [Doctoral dissertation, University of Kaiserslautern, Germany]. ProQuest Dissertations Publishing
22. Geletneky, K., Hajda J, Angelova AL, Leuchs B, Capper D, Bartsch AJ, Neumann JO, Schöning T, Hüsing J, Beelte B, Kiprianova I, Roscher M, Bhat R, von Deimling A, Brück W, Just A, Frehtman V, Löbhard S, Terletskaia-Ladwig E, Fry J, Jochims K, Daniel V, Krebs O, Dahm M, Huber B, Unterberg A, Rommelaere J. (2017) Oncolytic H-1 Parvovirus shows safety and signs of immunogenic activity in a first phase I/IIa glioblastoma trial. Mol Ther. 25, 2620-2634
23. Hajda J, Leuchs B, Angelova AL, Frehtman V, Rommelaere J, Mertens M, Pilz M, Kieser M, Krebs O, Dahm M, Huber B, Engeland CE, Mavratzas A, Hohmann N, Schreiber J, Jager D, Halama N, Sedlaczek O, Gaida MM, Daniel V, Springfeld C, Ungerechts G. (2021) Phase 2 Trial of Oncolytic H-1 Parvovirus Therapy Shows Safety and Signs of Immune System Activation in Patients With Metastatic Pancreatic Ductal Adenocarcinoma. Clin Cancer Res. 27, 5546-5556
24. Willemsen A, Zwart MP. (2019) On the stability of sequences inserted into viral genomes. Virus Evol. 5, vez045
25. Tattersall P, Bratton J. (1983) Reciprocal productive and restrictive virus cell interactions of immunosuppressive and prototype strains of minute virus of mice. J. Virol. 46, 944-955
26. Kestler, J., Neeb, B., Struyf, S., Van Damme, J., Cotmore, S. F., D'Abramo, A., Tattersall, P., Rommelaere, J., Dinsart, C. and Cornelis, J. J. (1999) cis requirements for the efficient production of recombinant DNA vectors based on autonomous parvoviruses. Hum Gene Ther. 10, 1619-1632
27. Leuchs B, Roscher M, Muller M, Kürschner K, Rommelaere J. (2016) Standardized large-scale H-1PV production process with efficient quality and quantity monitoring. J Virol Methods. 229, 48-59
28. Li J, Bonifati S, Hristov G, Marttila T, Valmary-Degano S, Stanzel S, Schnölzer M, Mougin C, Aprahamian M, Grekova SP, Raykov Z, Rommelaere J, Marchini A. (2013) Synergistic combination of valproic acid and oncolytic parvovirus H-1PV as a potential therapy against cervical and pancreatic carcinomas. EMBO Mol Med. 5, 1537-1555
29. Monaco G, Chen H, Poidinger M, Chen J, de Magalhães JP, Larbi A. (2016) flowAI: automatic and interactive anomaly discerning tools for flow cytometry data. Bioinformatics. 32, 2473-2480
30. McInnes L, Healy J, Melville J. (2018) UMAP: Uniform Manifold Approximation and Projection for Dimension Reduction. ArXiv e-prints
31. Köhler, G., Milstein, C. (1975) Continuous cultures of fused cells secreting antibody of predefined specificity. Nature. 256, 495-497
32. Masuda, T., Endo, M., Yamamoto, Y., Odagiri, H., Kadomatsu, T., Nakamura, T., Tanoue, H., Ito, H., Yugami, M., Miyata, K., Morinaga, J., Horiguchi, H., Motokawa, I., Terada, K., Morioka, M. S., Manabe, I., Iwase, H., Mizuta, H., & Oike, Y. (2015). ANGPTL2 increases bone metastasis of breast cancer cells through enhancing CXCR4 signaling. Scientific reports, 5, 9170
33. Feoktistova M, Geserick P, Leverkus M. (2016) Crystal Violet Assay for Determining Viability of Cultured Cells. Cold Spring Harb Protoc. 2016, pdb.prot087379
34. Cavo, M., Delle Cave, D., D'Amone, E. Giuseppe Gigli, G., Enza Lonardo, E., Loretta L. del Mercato, L. (2020) A synergic approach to enhance long-term culture and manipulation of MiaPaCa-2 pancreatic cancer spheroids. Sci Rep 10, 10192

## Claims

1. A H-1 parvovirus variant comprising a 114-nucleotide internal deletion and a 55-nucleotide repeated motif towards the right-end terminus in the presence of full length of right-end ITRs of wild-type H-1 parvovirus (PV).

2. The parvovirus variant of claim 1 comprising an in-frame, 114-nucleotide deletion from nucleotide 2022 to 2135 in the open reading frame encoding the nonstructural proteins NS1 and NS2, and a duplication of a 55-nucleotide from nucleotide 4828 to 4883 towards the right-end terminus in the wildtype H-1 PV genome of Figure 8.

3. An antibody, directed against the NS1 and/or NS2 protein of the parvovirus variant according to claim 1 or 2 **characterized in that** it does only bind to the variant protein having the deletion but not to the wild type protein.

4. Kit comprising:
(a) a parvovirus variant of claim 1 or 2,
(b) an antibody according to claim 3; and/or, optionally,
(c) conventional auxiliary agents, such as solvents, buffers, carriers, markers and controls,
wherein of components (a) to (c) one or more representatives can be present each.

5. A pharmaceutical composition containing (a) a parvovirus variant according to claim 1 or 2 or the antibody of claim 3 and (b) a pharmaceutically acceptable carrier.

6. Use of a parvovirus variant according to claim 1 or 2 or the antibody of claim 3 for the preparation of a pharmaceutical composition for the treatment of cancer.

7. The use of claim 6, wherein the cancer is a solid or hematological tumor.

8. The use of claim 7, wherein the solid tumor is a brain cancer, colon cancer, bladder cancer, liver cancer, breast cancer, kidney cancer, head/neck squamous cell carcinoma, lung cancer, malignant melanoma, ovarian cancer, pancreatic cancer, prostate cancer, renal cell cancer or stomach cancer and/or metastases of the tumor.

9. The use of claim 8, wherein the hematological tumor is acute lymphoblastic leukemia (ALL), acute myeloid leukemia (AML), chronic lymphocytic leukemia (CLL), chronic myeloid leukemia (CML), diffuse large B-cell lymphoma (DLBCL), EBV-positive DLBCL, primary mediastinal large B-cell lymphoma, T-cell(histiocyte)-rich large B-cell lymphoma, follicular lymphoma, Hodgkin's lymphoma (HL), mantle cell lymphoma (MCL), multiple myeloma (MM), myeloid cell leukemia-1 protein (Mcl-1), myelodysplastic syndrome (MDS), non-Hodgkin's lymphoma (NHL), or small lymphocytic lymphoma (SLL) and/or metastases thereof.

10. The parvovirus variant according to claims 1 or 2 for use in a method of treating cancer.

11. The parvovirus variant for the use of claim 10, wherein the cancer is a solid or hematological tumor.

12. The parvovirus variant for the use of claim 11, wherein the solid tumor is a brain cancer, colon cancer, bladder cancer, liver cancer, breast cancer, kidney cancer, head/neck squamous cell carcinoma, lung cancer, malignant melanoma, ovarian cancer, pancreatic cancer, prostate cancer, renal cell cancer or stomach cancer and/or metastases of the tumor.

13. The parvovirus variant of claim 11, wherein the hematological tumor is acute lymphoblastic leukemia (ALL), acute myeloid leukemia (AML), chronic lymphocytic leukemia (CLL), chronic myeloid leukemia (CML), diffuse large B-cell lymphoma (DLBCL); EBV-positive DLBCL, primary mediastinal large B-cell lymphoma, T-cell(histiocyte)-rich large B-cell lymphoma, follicular lymphoma, Hodgkin's lymphoma (HL), mantle cell lymphoma (MCL), multiple myeloma (MM), myeloid cell leukemia-1 protein (Mcl-1), myelodysplastic syndrome (MDS), non-Hodgkin's lymphoma (NHL), or small lymphocytic lymphoma (SLL) and/or metastases thereof.
